# EUROPEAN PATENT APPLICATION

(11) **EP 2 033 646 A1**
(43) Date of publication of application: **11.03.2009**
(21) Application number: 07790222.9
(22) Date of filing: 27.06.2007
(51) Int. Cl.: A61K 31/50, A61K 31/336, A61K 31/366, A61K 31/40, A61K 31/404, A61K 31/47, A61K 31/505, A61K 45/00, A61P 29/00

(54) **PROPHYLACTIC AND/OR THERAPEUTIC AGENT FOR RHEUMATOID ARTHRITIS**

(30) Priority: 29.06.2006 US 817091 P
(71) Applicant: Kowa Co., Ltd., Nagoya-shi, Aichi 460-8625 (JP)
(72) Inventor: TABUNOKI, Yuichiro, Higashikurume-shi, Tokyo 203-0051 (JP)
(74) Representative: Blodig, Wolfgang
(86) International application number: PCT/JP2007/000700
(87) International publication number: WO 2008/001499

(57) **Abstract**

A medicament having excellent therapeutic and prophylactic effects on rheumatoid arthritis is provided.

A prophylactic and/or therapeutic agent for rheumatoid arthritis, comprising
2-benzyl-5-(4-chlorophenyl)-6-[4-(methylthio)phenyl]-2H-pyr idazin-3-one and an HMG-CoA reductase inhibitor in combination.

## Description

### Technical Field

The present invention relates to a prophylactic and/or therapeutic agent for rheumatoid arthritis.

### Background Art

Rheumatoid arthritis is a disease in which inflammation accompanied by swelling or pain occurs in multiple joints and progresses over a long period of time, thereby irreversible deformation of joints and functional disorders leading to significant lowering of the quality of life (QOL). In Japan, there are patients corresponding in number to 0.6% of the total population, and 1% of the population of age over 30. In particular, in recent years, the number of elderly patients of rheumatism tends to increase along with the progress of the aged society.

The disease stages of rheumatoid arthritis can be classified into four stages, such as the "initial phase, " a stage in which although manifestation of j oint pain or arthritis exists, definite diagnosis of rheumatoid arthritis cannot be given yet; the "early phase," a stage in which definite diagnosis of rheumatoid arthritis can be given, but there are no or, if any, slight irreversible changes (the early phase rheumatoid arthritis generally refers to a stage after 1 to 2 years from the onset of the disease); the "advanced phase" in which irreversible changes emerge, and systemic symptoms such as fatigue, mild fever, and weight loss appear intensely; and the "late phase" in which inflammation in the joints is almost going to remission, but irreversible changes such as deformation and contracture still remain strong, with the predominant symptoms being pain and functional disorders. Thus, the therapeutic method varies depending on the stage of rheumatoid arthritis. With regard to the pathogenesis of rheumatoid arthritis, there have been reported research results that hereditary factors or acquired factors (infection) are involved with the disease, but because the causes have not been clarified, it is still impossible to achieve complete cure or prevention of the disease. Therefore, the goal of therapy under the present circumstances lies in achieving early diagnosis of rheumatoid arthritis, suppressing the inflammation caused by rheumatoid arthritis rapidly and maximally as much as possible, and thus preventing the emergence of any irreversible changes or else inhibiting progress thereof, thereby attempting an improvement of the QOL of patients from physical, mental and social aspects. Therefore, during the therapy, patients aregivensufficient explanation on the disease or the therapeutic methods for the disease, and then various means of treatment such as physical therapy, kinesitherapy, drug therapy, and surgical therapy are applied to the patients.

In drug therapy for rheumatoid arthritis, non-steroidal antiinflammatory drugs (NSAIDs), disease-modifying antirheumatic drugs (DMARDs) and steroid drugs are being used in the clinical field. Recently, biologics such as antibodies against proinflammatory cytokines are also used (Non-Patent Document 1).

DMARDs are classified, in terms of the mechanism of action, into immunomodulators and immunosuppressants, and among them, methotrexate or leflunomide is acknowledged to have a high anti-rheumatic effect. However, since both methotrexate and leflunomide can induce serious adverse effects including infection and interstitial pneumonia, these drugs have drawbacks such that not only it is necessary to strictly control the method of administration or dosage, but also sufficient investigation on the background of affection of the patient is required.

Furthermore, although biologics have potent anti-rheumatic effects, the biologics also have a drawback in the aspect of medical economics that they are expensive, as well as another drawback in the aspect of convenience that the route of administration thereof is limited to injection, and oral administration is impossible.

In many diseases such as rheumatoid arthritis, osteoarthritis, osteoporosis, inflammatory bowel disease, immune deficiency syndrome, septicemia, hepatitis, nephritis, ischemic diseases, insulin-dependent diabetes, arteriosclerosis, Parkinson's disease, Alzheimer's disease, and leukemia, stimulation of production of interleukin 1β (IL-1β), which is a proinflammatory cytokine, is observed. IL-1β is known to induce the synthesis of enzymes which are conceived to be associated with inflammation, such as collagenase, COX, and PLA2. IL-1β is also known to cause articular damage which is very simi lar to that caused by rheumatoid arthritis when intra-articularly injected to animals. Thus, IL-1β inhibitors are being studied and developed as therapeutic agents for inflammatory diseases, and there are known biological materials such as IL-1 receptor antagonist (Non-Patent Document 2), and low molecular weight compounds such as T-614 (Non-Patent Document 3), S-2474 (Non-Patent Document 4), 2-benzyl-5-(4-chlorophenyl)-6-[4-(methylthio)phenyl]-2H-pyr idazin-3-one (Patent Document 1), and FR133605 (Non-Patent Document 5).

Arteriosclerotic diseases are representative lesions occurring in the vascular system, while ischemic heart diseases such as myocardial infarction and angina pectoris, cerebral infarction, and arteriosclerosis obliterans are diseases with high lethality whose incidences are globally increasing. Arteriosclerosis is conceived to be predominantly caused by proliferation of cellular components represented by smooth muscle cells and of extracellular matrix, as well as by intimal thickening as a result of accumulation of lipids including cholesterol. However, it has also come to be seen as inflammation associated with macrophages or lymphocytes (Non-Patent Document 6) .

Currently, therapeutic agents for hyperlipidemia having an action of ameliorating the blood lipid concentration are being frequently used, for the purpose of preventing arteriosclerotic diseases. Among them, a series of drugs generically called statins, whose mechanism of action is based on the HMG-CoA reductase inhibitory action, are known to be the most potent therapeutic agents for hyperlipidemia. Furthermore, as for statins that are clinically used, there are known pravastatin sodium, simvastatin, fluvastatin sodium, atorvastatin calcium hydrate, pitavastatin calcium, and the like.

In recent years, it has become evident that statins, which are HMG-CoA reductase inhibitors, have anti-inflammatory actions, and it has been reported that administration of statins is effective in an inflammatory arthritis model (Non-Patent Document 7) or in patients suffering from rheumatoid arthritis (Non-Patent Document 8).

The therapeutic target of a therapeutic agent for hyperlipidemia lies in reduction of cholesterol in the blood, which is a causative factor of arteriosclerosis. Meanwhile, macrophages which play a central role in the formation of plaques that are causative of arteriosclerotic diseases, produce proinflammatory cytokines including IL-1β which induces inflammation, and thereby atheroma which acts as a trigger of arteriosclerosis is formed (Non-Patent Document 9). That is, inhibition of the production of IL-1β is conceived to be also effective for arteriosclerotic diseases which are the diseases targeted by the therapeutic agents for hyperlipidemia. Moreover, it has been reported that acquisition of rheumatoid arthritis, in which disease proinflammatory cytokines including IL-1β are deeply involved in the onset, is accompanied by a high risk of the onset and exacerbation of arteriosclerotic diseases (Non-Patent Document 10). Inaddition, it has also been reported that administration of statins results in the suppression of arteriosclerosis in patients with rheumatoid arthritis (Non-Patent Document 11), and that statins suppress the production of proinflammatory cytokines by macrophages (Non-Patent Document 12).

As discussed above, an HMG-CoA reductase inhibitor and an IL-1β inhibitor have been suggested to be each individually useful in the treatment of rheumatoid arthritis. However, it is still not known what effects would be generated if the two drugs were used in combination.
[Patent Document 1] WO99/25697
[Non-Patent Document 1] Arthritis & Rheumatism, 46, pp. 328-346, 2002
[Non-Patent Document 2] Arthritis & Rheumatism, 42, pp. 498-506, 1999
[Non-Patent Document 3] J. Pharmacobio-Dyn., 11, pp. 649-655, 1992
[Non-Patent Document 4] YAKUGAKU ZASSHI, 123, pp. 323-330, 2003
[Non-Patent Document 5] J. Rheumatol. , 23, pp. 1778-1783, 1996
[Non-Patent Document 6] N. Engl. J. Med. , 340, pp. 115-126, 1999
[Non-Patent Document 7] J. Immunol., 170, pp. 1524-1530, 2003
[Non-Patent Document 8] Arthritis & Rheumatism, 48 (suppl.), p. S666, 2003
[Non-Patent Document 9] Nature, 420, pp. 868-874, 2002
[Non-Patent Document 10] Arthritis & Rheumatism, 46, pp. 862-873, 2002
[Non-Patent Document 11] Ann. Rheum. Dis., 63, pp. 1571-1575, 2004
[Non-Patent Document 12] J. Clin. Invest., 100, pp. 2671-2679, 1997

### Disclosure of the Invention

### Problems to be Solved by the Invention

It is an object of the present invention to provide a medicament having excellent therapeutic and prophylactic effects on rheumatoid arthritis.

### Means for Solving the Problems

In view of the foregoing, the present inventor has devotedly conducted research, and has found that use in combination of 2-benzyl-5-(4-chlorophenyl)-6-[4-(methylthio)phenyl]-2H-pyr idazin-3-one, which is an IL-1β inhibitor, and an HMG-CoA reductase inhibitor results in a synergistically excellent arthritis suppressive effect, and the effect is potent to the extent that it is unpredictable from the respective effects of the individual drugs. Thus, the inventor completed the present invention.

Accordingly, the present invention provides a prophylactic and/or therapeutic agent for rheumatoid arthritis, comprising 2-benzyl-5-(4-chlorophenyl)-6-[4-(methylthio)phenyl]-2H-pyr idazin-3-one and an HMG-CoA reductase inhibitor in combination.

The present invention also provides a prophylactic and/or therapeutic agent for rheumatoid arthritis, comprising 2-benzyl-5-(4-chlorophenyl)-6-[4-(methylthio)phenyl]-2H-pyr idazin-3-one as an active ingredient, characterized by being administered in combination with an HMG-CoA reductaseinhibitor, for the purpose of prevention and/or treatment of rheumatoid arthritis.

The present invention also provides a prophylactic and/or therapeutic agent for rheumatoid arthritis, comprising an HMG-CoA reductase inhibitor as an active ingredient, characterized by being administered in combination with 2-benzyl-5-(4-chlorophenyl)-6-[4-(methylthio)phenyl]-2H-pyr idazin-3-one, for the purpose of prevention and/or treatment of rheumatoid arthritis.

The present invention also provides use of a combination of 2-benzyl-5-(4-chlorophenyl)-6-[4-(methylthio)phenyl]-2H-pyr idazin-3-one and an HMG-CoA reductase inhibitor, for the manufacture of a prophylactic and/or therapeutic agent for rheumatoid arthritis.

The present invention also provides use of 2-benzyl-5-(4-chlorophenyl)-6-[4-(methylthio)phenyl]-2H-pyr idazin-3-one, for the manufacture of a prophylactic and/or therapeutic agent for rheumatoid arthritis which is administered in combination with an HMG-CoA reductase inhibitor for the purpose of prevention and/or treatment of rheumatoid arthritis.

The present invention also provides use of an HMG-CoA reductase inhibitor, for the manufacture of a prophylactic and/or therapeutic agent for rheumatoid arthritis which is administered in combination with 2-benzyl-5-(4-chlorophenyl)-6-[4-(methylthio)phenyl]-2H-pyr idazin-3-one for the purpose of prevention and/or treatment of rheumatoid arthritis.

The present invention also provides a method for treating rheumatoid arthritis, comprising administering effective amounts of 2-benzyl-5-(4-chlorophenyl)-6-[4-(methylthio)phenyl]-2H-pyr idazin-3-one and an HMG-CoA reductase inhibitor.

### Effects of the Invention

The prophylactic and/or therapeutic agent for rheumatoid arthritis of the present invention can be orally administered, exhibits an excellent suppressive effect on arthritis with fewer adverse effects, and is not only useful in the prevention and treatment of rheumatoid arthritis, but also useful for patients with concurrent arteriosclerotic disease.

### Brief Description of the Drawings

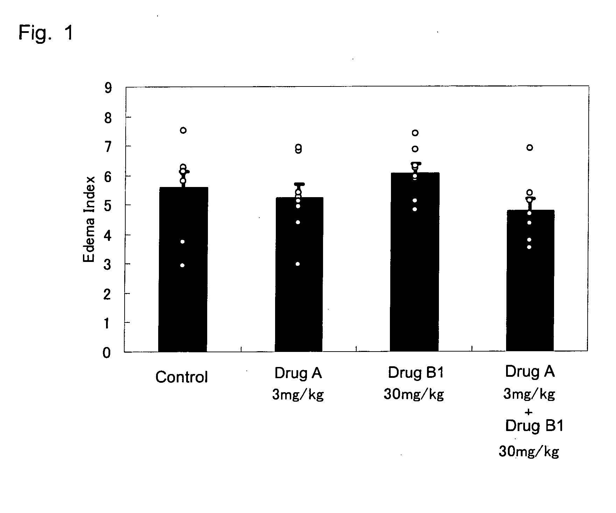

Fig. 1 is a diagram showing the Edema Indices of rats of a collagen-induced arthritis model in the case of combining the administration of 2-benzyl-5-(4-chlorophenyl)-6-[4-(methylthio)phenyl]-2H-pyr idazin-3-one (Drug A) at a dose of 3 mg/kg twice a day, and the administration of atorvastatin calcium hydrate (Drug B1) at a dose of 30 mg/kg once a day.

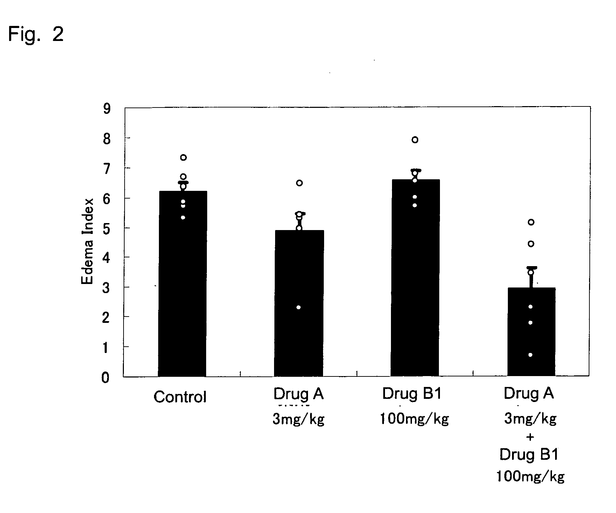

Fig. 2 is a diagram showing the Edema Indices of rats of a collagen-induced arthritis model in the case of combining the administration of 2-benzyl-5-(4-chlorophenyl)-6-[4-(methylthio)phenyl]-2H-pyr idazin-3-one (Drug A) at a dose of 3 mg/kg twice a day, and the administration of atorvastatin calcium hydrate (Drug B1) at a dose of 100 mg/kg once a day.

### Best Modes for Carrying Out the Invention

2-Benzyl-5-(4-chlorophenyl)-6-[4-(methylthio)phenyl]-2H-pyridazin-3-one (hereinafter, may also be described as Drug A), which is used as a prophylactic and/or therapeutic agent for rheumatoid arthritis of the present invention, may be produced through, for example, the method described in WO 99/25697, or a similar method. That is, p-chlorophenylacetic acid is reacted with thioanisole using a condensing agent such as polyphosphoric acid, to obtain 2-(4-chlorophenyl)-4'-(methylthio)acetophenone. The thus obtained 2-(4-chlorophenyl)-4'-(methylthio)acetophenone is reacted with a base such as potassium t-butoxide in tetrahydrofuran, and then ethyl bromoacetate is added thereto, to obtain ethyl 2-(4-chlorophenyl)-4-[4-(methylthio)phenyl]-4-oxobutanoate. The thus obtained ethyl 2-(4-chlorophenyl)-4-[4-(methylthio)phenyl]-4-oxobutanoate is reacted with hydrazine hydrate in ethanol, to obtain 5-(4-chlorophenyl)-6-[4-(methylthio)phenyl]-4,5-dihydro-2H-pyridazin-3-one. The thus obtained 5-(4-chlorophenyl)-6-[4-(methylthio)phenyl]-4,5-dihydro-2H-pyridazin-3-one is reacted with benzyl bromide in a solvent such as N,N-dimethylformamide, using a base such as potassium carbonate, to thereby yield 2-benzyl-5-(4-chlorophenyl)-6-[4-(methylthio)phenyl]-2H-pyr idazin-3-one.

The HMG-CoA reductase inhibitor (hereinafter, may also be described as Drug B), which is used as a prophylactic and/or therapeutic agent for rheumatoid arthritis of the present invention, includes all of so-called statins which have cholesterol synthesis inhibitory activities, and are known as therapeutic agents for hyperlipidemia. As the HMG-CoA reductase inhibitor used as the prophylactic and/or therapeutic agent for rheumatoid arthritis of the present invention, compounds having a 3,5-dihydroxyheptanoic acid or 3,5-dihydroxy-6-heptenoic acid structure, specifically those compounds described in JP-A-57-2240, JP-A-57-163374, JP-A-56-122375, JP-A-60-500015, JP-A-01-216974, JP-A-03-58967, JP-A-01-279866, JP-A-05-178841, and the like may be mentioned, while lactone derivatives and ring-opened lactone derivatives or salts thereof are all included. Further included are hydrates or solvates with pharmaceutically acceptable solvents of these compounds and salts thereof, and also all isomers thereof in the case where asymmetric carbon atoms are present in these compounds and in the case where these compounds have unsaturated bonds and stereoisomers thereof exist.

As preferred HMG-CoA reductase inhibitors according to the present invention, there may be mentioned statins, such as pravastatin
((+)-(3R,5R)-3,5-dihydroxy-7-[(1S,2S,6S,8S,8aR)-6-hydroxy-2 -methyl-8-[(S)-2-methylbutyryloxy]-1,2,6,7,8,8a-hexahydro-1 -naphthyl]heptanoic acid, JP-A-57-2240), salts thereof, or solvates of the compound or the salts (e.g., pravastatin sodium); simvastatin
((+)-(1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethy 1]-1-naphthyl 2,2-dimethylbutyrate, JP-A-56-122375) or solvates thereof; fluvastatin
((±)-(3R*,5S*,6E)-7-[3-(4-fluorophenyl)-1-(1-methylethyl)-1 H-indol-2-yl]-3,5-dihydroxy-6-heptenoic acid, JP-A-60-500015), salts thereof, or solvates of the compound or the salts (e.g., fluvastatin sodium); atorvastatin ((3R,5R)-7-[2-(4-fluorophenyl)-5-(1-methylethyl)-3-phenyl-4 -phenylaminocarbonyl-1H-pyrrol-1-yl]-3,5-dihydroxyheptanoic acid, JP-A-03-58967), salts thereof, or solvates of the compound or the salts (e.g., atorvastatin calcium hydrate) ; pitavastatin ((3R,5S,6E)-7-[2-cyclopropyl-4-(4-fluorophenyl)-3-quinolyl] -3,5-dihydroxy-6-heptenoic acid, JP-A-01-279866), salts thereof, or solvates of the compound or the salts (e.g., pitavastatin calcium); and rosuvastatin (7-[4-(4-fluorophenyl)-6-isopropyl-2-(N-methyl-N-methylsulf onylamino)pyrimidin-5-yl]-(3R,5S)-dihydroxy-(E)-6-heptenoic acid, JP-A-05-178841), salts thereof, orsolvatesof the compound and the salts. In particular, atorvastatin, salts thereof, or solvates of the compound or the salts (hereinafter, may also be described as Drug B1) are preferred. The HMG-CoA reductase inhibitors may be produced through the methods described in the above-mentioned patent publications, as well as known methods. Furthermore, atorvastatin calcium hydrate which is a hydrate of a salt of atorvastatin may also be a commercially available productsuch as a product manufactured by NFTZ Alchem International Co., Ltd.

The combination ratio of the amounts of incorporation or amounts of administration of Drug A and Drug B used as the prophylactic and/or therapeutic agent for rheumatoid arthritis of the present invention is preferably in the range of 100:1 to 1:300, more preferably 80:1 to 3:100, and particularly preferably 50:1 to 3:100, by mass, from the viewpoint of obtaining a particularly excellent synergistic effect.

The dosage form of the drug used as the prophylactic and/or therapeutic agent for rheumatoid arthritis of the present invention is not particularly limited, and can be appropriately selected according to the purpose of treatment. For example, oral administration by means of tablets, capsules, granules, film-coated drugs, powders, syrups and the like, or parenteral administration by means of injections, suppositories, inhalations, percutaneous drugs, eye drops, nose drops and the like may be mentioned, while oral administration is particularly preferred.

The prophylactic and/or therapeutic agent for rheumatoid arthritis of the present invention, formed by combining Drug A and Drug B, may be prepared such that the active ingredients, Drug A and Drug B, are made into separate preparations, and the preparations maybe administered simultaneously or at an interval. Alternatively, the prophylactic and/or therapeutic agent may also be administered as a pharmaceutical preparation containing both of the active ingredients. Furthermore, as for the prophylactic and/or therapeutic agent for rheumatoid arthritis of the present invention, which contains Drug A as an active ingredient and is administered in combination with Drug B for the purpose of prevention and/or treatment of rheumatoid arthritis, the preparation containing Drug A may be administered simultaneously or at an interval with the preparation containing Drug B. As for the prophylactic and/or therapeutic agent for rheumatoid arthritis of the present invention, which contains Drug B as an active ingredient and is administered in combination with Drug A for the purpose of prevention and/or treatment of rheumatoid arthritis, the preparation containing Drug B may be administered simultaneously or at an interval with the preparation containing Drug A.

A pharmaceutical preparation suitable for these dosage forms may be appropriately combined with pharmaceutically acceptable carriers, for example, excipients or bulking agents such as starches, lactose, sucrose, mannitol, and silicic acid; disintegrants such as agar, calcium carbonate, potato or tapioca starch, alginic acid, and particular complex silicates; binders such as hydroxypropylmethylcellulose, alginates, gelatin, polyvinylpyrrolidone, sucrose, and acacia; lubricants such as talc, calcium stearate, magnesium stearate, solid polyethylene glycols, sodium lauryl sulfate, and mixtures thereof; diluents such as lactose and corn starch; buffers such as organic acids (e.g. , citric acid, tartaric acid, and lactic acid), inorganic acids (e.g., phosphoric acid and hydrochloric acid), alkali hydroxides (e.g., sodium hydroxide and potassium hydroxide), and amines (e.g., triethanolamine, diethanolamine, and diisopropanolamine); antiseptics such as p-oxybenzoate esters and benzalkonium chloride; emulsifying agents such as anionic surfactants (e.g., calcium stearate, magnesium stearate, and sodium lauryl sulfate), cationic surfactants (e.g., benzalkonium chloride, benzethonium chloride, and cetylpyridinium chloride), and nonionic surfactants (e.g., glyceryl monostearate, sucrose fatty acid esters, polyoxyethylene hydrogenated castor oil, polyoxyethylene sorbitan fatty acid esters, polyoxyethylene fatty acid esters, and polyoxyethylene alkyl ethers); stabilizing agents such as sodium sulfite, sodium hydrogen sulfite, dibutylhydroxytoluene, butylhydroxyanisole, and EDTA. In addition, additives such as anodor-suppressor, adispersant, apreservative, andaflavoring agent may appropriately be used in accordance with needs.

In the present invention, the dosage of 2-benzyl-5-(4-chlorophenyl)-6-[4-(methylthio)phenyl]-2H-pyr idazin-3-one may be appropriately selected in accordance with the weight, age, gender, symptoms and the like of the patient, but typically, a daily dose for an adult may be 2 to 320 mg, and preferably 4 to 160 mg, per day. Furthermore, the dosage of the HMG-CoA reductase inhibitor may vary with the type, but a daily dose for an adult may be 1 to 60 mg per day. The administration may be carried out once a day, or in two or more divided portions a day.

### EXAMPLES

Hereinafter, the present invention will be more specifically described by way of Examples, but the present invention is not to be limited to these Examples.

### EXAMPLE 1

For the cases of combined administration and respective single drug administrations of 2-benzyl-5-(4-chlorophenyl)-6-[4-(methylthio)phenyl]-2H-pyr idazin-3-one and atorvastatin calcium hydrate, the effect of edema suppression in both hindlimbs was determined by the following method (rat collagen-induced arthritis model) (FDA, CBER, CDER, CDRH: Guidance for industry - Clinical development programs for drugs, devices, and biological products for the treatment of rheumatoid arthritis (RA)-.(1999)).

For the test animal, female Lewis rats (LEW/Crj) (obtained from Charles River Laboratories Japan, Inc.) were used.

For each 8-week-old LEW/Crj rat, the volume of a portion from the ankle to the toe tip of both hindlimbs (hereinafter, both hindlimbs volume) was measured by means of a plethysmometer for small animals (TK-101CMP, product of Unicom), and the total volume was taken as the both hindlimbs volume upon initiation of test (hereinafter, Pre value). This Pre value was used as an index to divide the rats into groups by randomized block design by the single variable, so that the values in the respective groups averaged out.

The collagen emulsion for sensitization that was used to induce arthritis in rats, was prepared by homogenizing a 0.3% Type II collagen solution (Collagen Research Center), Adjuvant Peptide (Peptide Institute, Inc.) and Adjuvant Incomplete Freund (DIFCO), using a Handy Micro Homogenizer (Microtec Nition, Co. , Ltd.) under ice-cooling. The collagen emulsion thus prepared was intracutaneously administered at 10 sites in the back of each rat at a dose of 0.1 mL/site, to perform initial sensitization of the rat. Seven days after the initial sensitization, 0.12 mL of the same collagen emulsion was intracutaneously administered to the root of tail in each rat, to perform booster sensitization.

Drug administration was conducted from the day after the initial sensitization through 26 days thereafter. The group for sole administration of 2-benzyl-5-(4-chlorophenyl)-6-[4-(methylthio)phenyl]-2H-pyr idazin-3-one (Drug A) was orally administered with the drug at 3 mg/kg, twice a day, in the morning (9:00 to 11:00) and in the evening (15:30 to 17:30). The group for sole administration of atorvastatin calcium hydrate (DrugB1) was orally administered with the drug at 30 or 100 mg/kg, once a day, in the afternoon (11:30 to 13:30). On the other hand, the group for combined administration of Drug A and Drug B1 was orally administered with Drug A at 3 mg/kg in the morning (9:00 to 11:00) and in the evening (15:30 to 17:30), and with Drug B1 at 30 or 100 mg/kg in the afternoon (11:30 to 13:30).
14, 18, 22 and 26 days after the initial sensitization, the both hindlimbs volume was measured again for each rat, and the difference between each of the values and the Pre value was determined to calculate the edema volume for both hindlimbs. The sum of the edema volumes for both hindlimbs measured 14, 18, 22 and 26 days after the initial sensitization was calculated as Edema Index, and this was taken as an index for the effects of the drugs.

Table 1 and Fig. 1 present the Edema Indices of the group for sole administration of 3 mg/kg of Drug A, the group for sole administration of 30 mg /kg of Drug B1, and the group for combined administration of both drugs. Furthermore, Table 2 and Fig. 2 present the Edema Indices of the group for sole administration of 3 mg/kg of Drug A, the group for sole administration of 100 mg /kg of Drug B1, and the group for combined administration of both drugs. The Edema Index is expressed in the average value ± standard error of 8 rats in each group. Furthermore, the decrease rate is indicated as ((X - Y)/X) x 100 (X: average Edema Index for control group, Y: agerage Edema Index for each group).

**[Table 1]**

| Test drug | Edema Index | Decrease rate (%) | Relative index |
|---|---|---|---|
| Control group | 5.59±0.53 | | 1.000 |
| Group for sole administratio n of 3 mg/kg of Drug A | 5.23±0.45 | 6.5 | 0.935 |
| Group for sole administration of 30 mg/kg of Drug B1 | 6.07±0.30 | -8.6 | 1.086 |
| Group for combined administratio n of Drug A and Drug B1 | 4.80±0.37 | 14.2 | 0.858 |

| | | | |
|---|---|---|---|
| Remark 1) Product of relative indices of the groups for sole administration: 0.935 × 1.086 = 1.015 Remark 2) The Edema Index is expressed in the average value ± standard error of 8 rats in each group. | | | |

**[Table 2]**

| Test drug | Edema Index | Decrease rate (%) | Relative index |
|---|---|---|---|
| Control group | 6.20±0.30 | | 1.000 |
| Group for sole administratio n of 3 mg/kg of Drug A | 4.89±0.57 | 21.0 | 0.790 |
| Group for sole administration of 100 mg/kg of Drug B1 | 6.57±0.31 | -6.0 | 1.060 |
| Group for combined administratio n of Drug A and Drug B1 | 2.95±0.69 | 52.4 | 0.476 |

| | | | |
|---|---|---|---|
| Remark 1) Product of relative indices of the groups for sole administration: 0.790 × 1.060 = 0.837 Remark 2) The Edema Index is expressed in the average value ± standard error of 6 rats in each group. | | | |

Although Drug B1 did not show any effects on edema in the current model (Edema Index) at the doses of 30 and 100 mg/kg, combined administration of Drug A and Drug B1 strongly decreased the Edema Index. Furthermore, the relative index of the Edema Index for the group of combined administration of Drug A and Drug B1 was smaller than the product of the relative indices of the respective groups for sole administration of the drugs, and clear synergistic effects due to the combined use were recognized. As such, the effects of the combined use of Drug A and Drug B on the prevention and treatment of rheumatoid arthritis were shown to be far exceeding the therapeutic effects expected from the respective effects of sole administration of the drugs.

## Claims

1. A prophylactic and/or therapeutic agent for rheumatoid arthritis, comprising
2-benzyl-5-(4-chlorophenyl)-6-[4-(methylthio)phenyl]-2H-pyr idazin-3-one and an HMG-CoA reductaseinhibitor in combination.

2. A prophylactic and/or therapeutic agent for rheumatoid arthritis, comprising
2-benzyl-5-(4-chlorophenyl)-6-[4-(methylthio)phenyl]-2H-pyr idazin-3-one as an active ingredient, wherein the prophylactic and/or therapeutic agent is administered in combination with an HMG-CoA reductase inhibitor for the purpose of prevention and/or treatment of rheumatoid arthritis.

3. A prophylactic and/or therapeutic agent for rheumatoid arthritis, comprising an HMG-CoA reductase inhibitor as an active ingredient, wherein the prophylactic and/or therapeutic agent is administered in combination with
2-benzyl-5-(4-chlorophenyl)-6-[4-(methylthio)phenyl]-2H-pyr idazin-3-one for the purpose of prevention and/or treatment of rheumatoid arthritis.

4. The prophylactic and/or therapeutic agent for rheumatoid arthritis according to any one of claims 1 to 3, wherein the HMG-CoA reductase inhibitor is pravastatin, a salt thereof, or a solvate of the compound or the salt; simvastatin or a solvate thereof ; fluvastatin, a salt thereof , or a solvate of the compound or the salt; atorvastatin, a salt thereof, or a solvate of the compound or the salt; pitavastatin, a salt thereof, or a solvate of the compound or the salt; or rosuvastatin, a salt thereof, or a solvate of the compound or the salt.

5. The prophylactic and/or therapeutic agent for rheumatoid arthritis according to any one of claims 1 to 4, wherein the HMG-CoA reductase inhibitor is atorvastatin, a salt thereof, or a solvate of the compound or the salt.

6. The prophylactic and/or therapeutic agent for rheumatoid arthritis according to any one of claims 1 to 5, wherein the dosage form is a preparation for oral administration.

7. The prophylactic and/or therapeutic agent for rheumatoid arthritis according to any one of claims 1 to 6, wherein
2-benzyl-5-(4-chlorophenyl)-6-[4-(methylthio)phenyl]-2H-pyr idazin-3-one and the HMG-CoA reductase inhibitor are respectively prepared into separate preparations.

8. Use of a combination of
2-benzyl-5-(4-chlorophenyl)-6-[4-(methylthio)phenyl]-2H-pyr idazin-3-one and an HMG-CoA reductase inhibitor, for the manufacture of a prophylactic and/or therapeutic agent for rheumatoid arthritis.

9. Use of
2-benzyl-5-(4-chlorophenyl)-6-[4-(methylthio)phenyl]-2H-pyr idazin-3-one, for the manufacture of a prophylactic and/or therapeutic agent for rheumatoid arthritis which is administered in combination with an HMG-CoA reductase inhibitor for the purpose of prevention and/or treatment of rheumatoid arthritis.

10. Use of an HMG-CoA reductase inhibitor, for the manufacture of a prophylactic and/or therapeutic agent for rheumatoid arthritis which is administered in combination with 2-benzyl-5-(4-chlorophenyl)-6-[4-(methylthio)phenyl]-2H-pyr idazin-3-one for the purpose of prevention and/or treatment of rheumatoid arthritis.

11. The use according to any one of claims 8 to 10, wherein the HMG-CoA reductase inhibitor is pravastatin, a salt thereof, or a solvate of the compound or the salt; simvastatin or a solvate thereof ; fluvastatin, a salt thereof , or a solvate of the compound or the salt; atorvastatin, a salt thereof, or a solvate of the compound or the salt; pitavastatin, a salt thereof, or a solvate of the compound or the salt; or rosuvastatin, a salt thereof, or a solvate of the compound or the salt.

12. The use according to any one of claims 8 to 11, wherein the HMG-CoA reductase inhibitor is atorvastatin, a salt thereof, or a solvate of the compound or the salt.

13. The use according to any one of claims 8 to 12, wherein the dosage form is a preparation for oral administration.

14. The use according to any one of claims 8 to 13, wherein 2-benzyl-5-(4-chlorophenyl)-6-[4-(methylthio)phenyl]-2H-pyr idazin-3-one and the HMG-CoA reductase inhibitor are respectively prepared into separate preparations.

15. A method for treatment of rheumatoid arthritis, the method comprising administering effective amounts of
2-benzyl-5-(4-chlorophenyl)-6-[4-(methylthio)phenyl]-2H-pyr idazin-3-one and an HMG-CoA reductase inhibitor.

16. The method for treatment according to claim 15, wherein the HMG-CoA reductase is pravastatin, a salt thereof, or a solvate of the compound or the salt; simvastatin or a solvate thereof; fluvastatin, a salt thereof, or a solvate of the compound or the salt; atorvastatin, a salt thereof, or a solvate of the compound or the salt; pitavastatin, a salt thereof, or a solvate of the compound or the salt; or rosuvastatin, a salt thereof, or a solvate of the compound or the salt.

17. The method for treatment according to claim 15 or 16, wherein the HMG-CoA reductase inhibitor is atorvastatin, a salt thereof, or a solvate of the compound or the salt.

18. The method for treatment according to any one of claims 15 to 17, wherein the means for administration is oral administration.

19. The method for treatment according to any one of claims 15 to 18, wherein
2-benzyl-5-(4-chlorophenyl)-6-[4-(methylthio)phenyl]-2H-pyr idazin-3-one and the HMG-CoA reductase inhibitor are respectively prepared into separate preparations.
